# EUROPEAN PATENT APPLICATION

(11) **EP 4 722 235 A1**
(43) Date of publication of application: **08.04.2026**
(21) Application number: 24810470.5
(22) Date of filing: 23.05.2024
(51) Int. Cl.: C07K 14/55, A61K 38/00, A61K 38/20, A61P 35/00, C12N 5/0783

(54) **POLYPEPTIDE, AND CONJUGATE AND USE THEREOF**

(30) Priority: 24.05.2023 CN 202310593255
(71) Applicant: Nantong Yichen Biopharma. Co. Ltd., Nantong, Jiangsu 226002 (CN)
(72) Inventor: WANG, Feng, Nantong, Jiangsu 226002 (CN); ZHANG, Yuhan, Nantong, Jiangsu 226002 (CN); CHEN, Chen, Nantong, Jiangsu 226002 (CN)
(74) Representative: Zacco GmbH
(86) International application number: PCT/CN2024/095008
(87) International publication number: WO 2024/240233

(57) **Abstract**

The present invention provides a polypeptide, its conjugate, and applications thereof. The polypeptide comprises the amino acid sequence as shown in SEQ ID NO: 1, wherein the amino acid represented by X at position 14 and/or position 39 is capable of conjugating with a modifier and decoupling from the modifier in the tumor microenvironment to release the polypeptide. The binding capability of the polypeptide without the conjugated modifier to CD122 is designated as the first binding capability, while the binding capability of the polypeptide with the conjugated modifier to CD122 is designated as the second binding capability. The ratio of the first binding capability to the second binding capability is denoted as n, wherein n ≥ 5. Due to the masking effect of the modifier, the conjugate avoids activating NK cells and CD8+ T cells in peripheral circulation. Under the action of tumor-specific enzymes, the polypeptide decouples from the conjugate, restoring its activation of the IL-2Rβγ receptor and enabling targeted cytotoxic effects in tumor.

## Description

### Cross-reference to related application

The present application claims the benefit of priority to Chinese Patent Application No. 202310593255.2 filed to the China National Intellectual Property Administration on May 24, 2023 and entitled "Polypeptide and Its Conjugate, and Uses Thereof", the content of which is hereby incorporated by reference in its entirety.

### Technical Field

The present invention relates to the field of genetic engineering, and in particular to a polypeptide, its conjugate thereof, and applications thereof.

### Background

With the aging of the population, tumor has become one of the important factors affecting the global mortality. Advances in biology have positioned immune-based cancer therapies as a new breakthrough. The complex immune system comprises immune organs, immune cells, and immune molecules. Immune-based treatments include CAR-T cell therapy based on immune cells; however, due to their regulatory roles in immune cells, as well as the multifaceted, cross-functional and selective characteristics of their functions, immune molecules have emerged as the first choice for scientist to transform and utilize. To date, the FDA has approved 11 cancer immunotherapies, the most of which are monoclonal antibody against immune checkpoint inhibitors such asCTLA-4, PD-1/PD-L1. Due to the limitations of CAR-T in solid tumors and immune checkpoint inhibitors in "cold" tumors, the used of chemically synthesized small molecules or cytokines that directly modulate the tumor microenvironment and regulate adaptive immune pathways can achieve a wider broader and more comprehensive effects of treating tumors. In November 1984, a patient with metastatic melanoma received high-dose IL-2 treatment for several months, and all tumors disappear throughout his body, and did not recur for the next-29 years. This was the first time that the importance of activating immune cells through immune molecules in eliminating tumors was demonstrated. Therefore, the FDA approved high-dose IL-2 for the treatment of advanced renal cell carcinoma and malignant melanoma in 1992 and 1998, which also trigger a wave of research on IL-2.

IL-2, as a pleiotropic cytokine activated by antigens, regulates the differentiation and development of various lymphocytes and plays a vital role in immune activation and the maintenance of immune homeostasis. IL-2 exerts its function by binding to its receptor and initiating downstream signaling. The IL-2 receptor consists of three subunits: IL-2Rα (CD25), IL-2Rβ (CD122), and IL-2Rγ (CD132). The medium-affinity(Kd≈10⁻⁹ M) dimeric receptor formed by the β and γ subunits activates the transcription of target genes by activating STAT5, PI3K-AKT, and MAPK pathways; CD25, which has a lower affinity for IL-2 (Kd≈10⁻⁸ M), can capture IL-2, and then present it to the dimer receptor in trans, thereby forming a high-affinity trimeric receptor (Kd≈ 10⁻¹¹ M) to achieve signal transduction, of which CD25 is not essential for signaling. Due to the affinity difference between dimeric and trimeric receptors, low concentrations of IL-2 preferentially bind to Treg cells expressing trimeric receptors, thereby exhibiting immunosuppressive activity, while in high concentrations of IL-2 can engage dimeric receptors predominantly on CD8⁺T and NK cells, exhibiting immunostimulatory activity.

As a key cytokine regulating Treg cells, low-dose IL-2 therapy (0.5-1 million IU/m²) is critical for treating autoimmune diseases like vasculitis, inflammatory myopathy, and systemic lupus erythematosus. To achieve anti-tumor effects, the use of dose up to 600,000 IU/kg not only substantially increases the manufacturing cost but also induces toxic side effects such as vascular leak syndrome (VLS), pulmonary interstitial infiltration, and liver cell damage. To avoid Treg activation by low doses of IL-2 and toxicity cased by high doses, current modifications use site-directed mutations (e.g., superkine IL-2), non-site-specific PEG conjugation (e.g., NKTR-214), or site-specific PEG conjugation (e.g., THOR-707) to either attenuate or enhance CD25 affinity, thereby altering the bias of IL-2 toward dimeric receptor binding.

However, the above IL-2-based modifications all have the problem of extremely low yields due to the instability of natural proteins, and cannot completely block CD25. IL-2 mutants in prior art, such as Neo-2/15, enhancing the binding affinity for the β subunit (CD122 unit) while eliminating the CD25 affinity, achieving selective activation of CD8⁺T cells and NK cells without affecting Tregs. Nevertheless, due to their increased affinity for CD122, these mutants lead to excessive NK cell activation, making safety profiles and therapeutic windows major obstacles for their clinical application. Moreover, as small proteins, the IL-2 mutants in prior art have short half-lives. In mouse models, daily dosing is required to ensure the antitumor efficacy, and such high-frequency dosing significantly compromises patient's compliance.

With growing demands for clinical therapeutics, there is an urgent need to optimize dosing frequency and dosage levels. Thus, a key objective is to extend the half-life of IL-2 mutants while reducing their toxicity.

### Summary of the Invention

The primary objective of the present invention is to provide a polypeptide, its conjugate, and applications to address the issue in prior art where IL-2 mutants, due to their high affinity for CD122 (IL-2Rβ subunit) in peripheral circulation, activate NK cells and/or CD8⁺T cells, leading to systemic toxic side effects.

To achieve the above objective, according to the first aspect of the present invention, a polypeptide is provided. The polypeptide has an amino acid sequence as set forth in SEQ ID NO: 1, wherein the amino acid represented by X at position 14 and/or position 39 has the following characteristics: it is capable of conjugating with a modifier group and decoupling from the modifier in the tumor microenvironment to release the polypeptide; wherein the binding ability of the unconjugated polypeptide to CD122 is defined as the first binding ability, the binding ability of the conjugated polypeptide to CD122 is defined as the second binding ability, and the ratio of the first binding ability to the second binding ability is defined as n, where n ≥ 5.

Furthermore, the amino acid represented by X at position 14 and/or 39 is any amino acid containing a sulfhydryl side chain; preferably, the amino acid represented by X at position 14 and/or 39 forms an intramolecular covalent bond directly or via a linker; preferably, the linker comprises mercaptopyridine; preferably, the amino acid represented by X at position 14 and/or 39 comprise cysteine.

To achieve the above objective, according to the second aspect of the present invention, a polypeptide conjugate is provided. The polypeptide conjugate comprises a polypeptide and a modifier conjugated to the polypeptide, wherein the polypeptide has an amino acid sequence as set forth in SEQ ID NO: 1, and the amino acid represented by X at position 14 and/or position 39 has the following characteristics: it is capable of conjugating with the modifier and decoupling from the modifier in the tumor microenvironment to release the polypeptide; wherein the binding ability of the unconjugated polypeptide to CD122 is defined as the first binding ability, the binding ability of the conjugated polypeptide to CD122 is defined as the second binding ability, and the ratio of the first binding ability to the second binding ability is defined as n, where n ≥ 5.

Furthermore, the modifier comprises a polymer or a fatty acid; preferably, the polymer comprises PEG; preferably, the fatty acid comprises an unsaturated fatty acid or a saturated fatty acid; preferably, the unsaturated fatty acid or saturated fatty acid is selected from fatty acids having 6 to 22 carbon atoms; preferably, the saturated fatty acid is any one selected from the followings: 17-carboxyheptadecanoic acid, 19-carboxynonadecanoic acid, 4-[N-(2-carboxyethyl)-N-(15-carboxypentadecanoyl)aminomethyl]benzoic acid, or [2-(2-{2-[2-(2-{2-[4-(17-carboxyheptadecanoylamino)-4(S)-carboxybutyrylamino]ethoxy}ethoxy)ac etylamino]ethoxy}ethoxy)acetic acid.

Furthermore, the amino acid represented by X at position 14 and/or position 39 of the polypeptide is any amino acid containing a sulfhydryl side chain; preferably, the modifier is conjugated to the sulfhydryl side chain of the amino acid at position 14 and/or position 39 of the polypeptide by a disulfide bond; preferably, the modifier is conjugated to the sulfhydryl side chain on the amino acid at position 14 and/or position 39 of the polypeptide by a mercaptorpyridine; preferably, the modifier is PEG; preferably, the amino acid represented by X at position 14 and/or position 39 of the polypeptide is cysteine.

In order to achieve the above object, according to the third aspect of the present invention, a DNA molecule is provided which encodes the polypeptide in the above polypeptide or polypeptide conjugate.

In order to achieve the above object, according to the fourth aspect of the present invention, a recombinant plasmid is provided, wherein the recombinant plasmid is connected to the above DNA molecule.

In order to achieve the above object, according to the fifth aspect, a host cell is provided, into which the above recombinant plasmid is transformed.

In order to achieve the above object, according to the sixth aspect, there is provided a use of the above polypeptide or the above polypeptide conjugate in the preparation of a drug for specifically activating CD8⁺ T cells and/or NK cells in a tumor microenvironment.

Furthermore, the above-mentioned use comprises use in the preparation of a drug for promoting immunity.

Furthermore, the above-mentioned use comprises use in the preparation of a drug for preventing and/or treating a solid tumor or a hematologic tumor; preferably, the solid tumor is bladder cancer, bone cancer, brain cancer, breast cancer, colorectal cancer, esophageal cancer, eye cancer, head and neck cancer, kidney cancer, lung cancer, melanoma, ovarian cancer, pancreatic cancer, or prostate cancer; preferably, hematologic tumor is chronic lymphocytic leukemia, small lymphocytic lymphoma, follicular lymphoma (FL), diffuse large B-cell lymphoma (DLBCL), mantle cell lymphoma, Waldenström's macroglobulinemia, multiple myeloma, extranodal marginal zone B-cell lymphoma, nodal marginal zone B-cell lymphoma, Burkitt lymphoma, non-Burkitt high-grade B-cell lymphoma, primary mediastinal large B-cell lymphoma, immunoblastic large cell lymphoma, precursor B-lymphoblastic lymphoma, B-cell prolymphocytic leukemia, lymphoplasmacytic lymphoma, splenic marginal zone lymphoma, plasma cell myeloma, plasmacytoma, mediastinal (thymic) large B-cell lymphoma, intravascular large B-cell lymphoma, primary effusion lymphoma, or lymphomatoid granulomatosis.

By applying the technical solution of the present invention, a polypeptide with the amino acid sequence of SEQ ID NO: 1 is provided, wherein X at positions 14 and Y at position 39 are capable of conjugating with a modifier having certain steric hindrance. This polypeptide retains the property of not binding to CD25 (IL-2Rα subunit) and, due to its reduced binding affinity to CD122, avoids the toxicity observed in prior art IL-2 mutants-such as abnormal elevation of cytokines or abnormal reduction in body weight-resulting from excessive activation of NK cells and CD8⁺T cells in peripheral circulation. Furthermore, the polypeptide provides specific sites for conjugation, thereby facilitating the preparation of subsequent conjugates.

In a preferred embodiment, the polypeptide is conjugated with a modifier. The resulting polypeptide conjugate exhibits substantially reduced binding capability to CD122 (IL-2Rβ subunit) due to the masking effect of the modifier, thereby avoiding activation of NK cells and CD8⁺T cells in the peripheral circulation. Within the tumor microenvironment, tumor microenvironment-specific enzymes can selectively cleave the conjugate and release the polypeptide. The released polypeptide restores its ability to activate the IL-2Rβγ receptor, specifically activating NK cells and T cells within the tumor microenvironment to exert tumor-specific cytotoxicity. This confines the specific cytotoxicity to the tumor site, enhancing both specificity and efficacy while avoiding peripheral toxic side effects. Moreover, conjugation of modifier with steric hindrance significantly extends the in vivo half-life of the polypeptide, which in turn helps reduce clinical dosing frequency and improves patient compliance. This approach enables achieving desirable therapeutic outcomes with lower dosing frequency and reduced dosage.

### Brief Description of the Drawings

The drawings constituting a part of this application are used to provide a further understanding of the present invention. The exemplary embodiments of the present invention and their descriptions are used to explain the present invention and do not constitute an improper limitation of the present invention. In the drawings:
Figure 1 shows the structure diagram of IL-2 and its receptor in Example 1 of the present invention.
Figure 2 shows the proliferative activity on CTLL2 cells of polypeptides with single-site cysteine mutations at different positions in Example 1 of the present invention.
Figure 3 shows the proliferative activity of polypeptide (14C) on MO7E cells in Example 2 of the present invention.
Figure 4 shows the proliferative activity of polypeptide (39C) on MO7E cells in Example 2 of the present invention.
Figure 5 shows a schematic diagram of the reaction between OPSS-PEG and cysteine in Example 2 of the present invention.
Figure 6 shows the proliferative activity of polypeptide (39C)-PEG on CTLL2 and MO7E cells in Example 2 of the present invention.
Figure 7 shows the SDS-PAGE results of CC-PEG in Example 2 of the present invention.
Figure 8 shows the size-exclusion chromatography result of CC-PEG in Example 2 of the present invention
Figure 9 shows the decoupling of CC-PEG in 0.3 mM GSH over time in Example 3 of the present invention.
Figure 10 shows the decoupling of CC-PEG in 3 mM GSH over time in Example 3 of the present invention.
Figure 11 shows the proliferative activity of CC on CTLL2 and MO7E cells in Example 3 of the present invention.
Figure 12 shows the stability of CC-PEG at different time points in plasma in example 4 of the present invention.
Figure 13 shows the proliferative activity of Neo-2/15, CC-PEG, and CC-PEG+GSH on CTLL2/MO7E cells in Example 5 of the present invention.
Figure 14 shows the effects Q1D administration of Neo-2/15 (Q1D) and Q4D administration of CC-PEG (0.15 mg/kg) on the body weight of mice and the anti-tumor effect in Example 7 of the present invention.
Figure 15 shows the analysis of the proportion of lymphocytes in different tissues when mice were sacrificed after administration of Neo-2/15 at Q1D and CC-PEG 0.15 mg/kg at Q4D in Example 7 of the present invention.
Figure 16A shows the effects of different doses of CC-PEG administered Q4D on the body weight of mice in Example 8 of the present invention.
Figure 16B shows the effect of different doses of CC-PEG administered Q4D on the percentage of weight change in mice in Example 8 of the present invention.
Figure 17 shows the effects of different doses of CC-PEG administered Q4D on the anti-tumor effect of mice in Example 8 of the present invention.
Figure 18A shows the effect of Q1D administration of Neo-2/15 and Q4D administration of CC-PEG 1 mg/kg on the body weight of mice in Example 9 of the present invention.
Figure 18B shows the effect of Q1D administration of Neo-2/15 and Q4D administration of CC-PEG 1 mg/kg on the percentage of weight change in mice in Example 9 of the present invention.
Figure 19 shows the effects of Q1D administration of Neo-2/15 and Q4D administration of CC-PEG 1 mg/kg on the anti-tumor effect in mice in Example 9 of the present invention
Figure 20 shows the results of Q1D administration of Neo-2/15 and Q4D administration of CC-PEG 1 mg/kg inducing IFN-γ production in mice in Example 9 of the present invention.

### Detailed Description

It is noted that embodiments of this application and their features may be combined with each other without conflict. The present invention will be described in detail below in conjunction with the embodiments.

As mentioned in the background, IL-2, as a pleiotropic cytokine activated by antigens, can regulate lymphocyte differentiation and development and plays a critical role in immune activation and maintaining immune homeostasis. Existing technologies have developed IL-2 mutants that do not bind to CD25 binding while exhibiting enhanced affinity for the dimeric receptor. Although such mutants avoid the potential immunosuppression caused by Treg activation, their high affinity for the β subunit pose potential toxic risks to cells. Therefore, the inventors of the present application developed a polypeptide conjugate with extended half-life, which can specifically act in tumor tissues and has reduced toxicity.

In a first typical embodiment of the present application, a polypeptide is provided, which has an amino acid sequence as set forth in SEQ ID NO: 1, wherein the amino acid represented by X at position 14 and/or position 39 has the following characteristics: it is capable of conjugating with a modifier and can be uncoupled from the modifier in the tumor microenvironment to release the polypeptide; wherein the binding ability of the unconjugated polypeptide to CD122 is defined as the first binding ability, the binding ability of the conjugated polypeptide to CD122 is defined as the second binding ability, and the ratio of the first binding ability to the second binding ability is defined as n, where n ≥ 5.

SEQ ID NO：1：

PKKKIQLHAEHALXDALMILNIVKTNSPPAEEKLEDAXNFELILEEIARLFESGDQKDEA
EKAKRMKEWMKRIKTTASEDEQEEMANAIITILQSWIFS。

The above polypeptide does not bind to CD25, thereby avoiding potential immunosuppression caused by Treg activation. At the same time, its binding capacity to CD122 (IL-2Rβ subunit) is significantly reduced, avoiding the high affinity of IL-2 mutants to CD122 seen in prior art, which could otherwise activates peripheral immune cells such as CD8⁺T cells and NK cells. Furthermore, this polypeptide provides conjugation sites for subsequent conjugates preparation. That is, any one or both of the amino acid(s) at positions 14 and 39 can be coupled to the modifier, which can further increase steric hindrance, enabling the development of conjugate-based therapeutics for clinical application. To ensure that the peptide with conjugated modifiers at positions 14 and/or 39 specifically functions within the tumor microenvironment by releasing the modifiers, while remaining inactive in the peripheral blood environment, a conjugation method and modifier can be selected such that the peptide's binding affinity to CD122 decreases by more than fivefold after conjugation.

The amino acid represented by X in the polypeptide can conjugate with modifier to further enhance steric hindrance. Depending on the type of modifier, the chemical bond formed with the polypeptide may vary, and accordingly, the amino acid represented by X differs, so that the coupled modifier can be specifically cleaved in the tumor microenvironment. In a preferred embodiment, X at position 14 and/or 39 is any amino acid containing a sulfhydryl group in its side chain. The specific type of sulfhydryl-containing amino acid is not limited-X may be other natural or unnatural amino acids or modified amino acids suitable for chemical conjugation. In a particularly preferred embodiment, X at position 14 and/or 39 is cysteine (i.e., polypeptide (14C), polypeptide (39C), or polypeptide (14C+39C), designated CC).

The formation of intramolecular chemical bonds within the polypeptide can also contribute to the subsequent preparation of polypeptide conjugates with substantial steric hindrance, thereby avoiding Treg activation. In a preferred embodiment, the amino acid represented by X at position 14 and/or 39 forms an intramolecular covalent linkage directly or via a linker. Any linker capable of forming such an intramolecular covalent linkage is applicable in the present application. In a preferred embodiment, the linker comprises a thiol-pyridyl group. This linker contains a sulfhydryl group capable of forming a disulfide bond with the sulfhydryl side chain of the amino acid represented by X at position 14 and/or 39 of the polypeptide. This covalent linkage can be specifically cleaved in the tumor microenvironment without affecting the intrinsic function of the polypeptide.

In a second typical embodiment of the present application, a polypeptide conjugate is provided. The polypeptide conjugate comprises a polypeptide and a modifier conjugated to the polypeptide, wherein the polypeptide has an amino acid sequence as set forth in SEQ ID NO: 1, and the amino acid represented by X at position 14 and/or position 39 has the following characteristics: it is capable of conjugating with the modifier and decoupling from the modifier in the tumor microenvironment to release the polypeptide; wherein the binding ability of the unconjugated polypeptide to CD122 is defined as the first binding ability, the binding ability of the conjugated polypeptide to CD122 is defined as the second binding ability, and the ratio of the first binding ability to the second binding ability is defined as n, where n ≥ 5.

The polypeptide conjugates are coupled on the basis of the polypeptides, and while retaining the ability of the polypeptide not to bind CD25, it partially or completely blocked its biding to CD122 (IL-2Rβ subunit). Due to the steric hindrance from the modifier, the conjugated cannot activate immune cell such as CD8⁺T and NK cells in peripheral circulation. However, they become active in tumor tissues after being specifically cleaved by enzymes in the tumor microenvironment, achieving tumor-specific functionality. This significantly reduces the risk of systemic toxicity associated with high CD122 binding affinity of IL-2 mutants in prior art. Unlike prior-art IL-2 mutants that significantly increase the proportion of NK cell in peripheral blood and may induce inflammation, the conjugates enhances lymphocyte infiltration in tumor tissues, significantly increase intratumoral NK and CD8⁺T cell populations, thereby confining its tumor killing effect to the tumor site. Additionally, the half-life of the conjugate is extended, reducing the high dosing frequency of daily administration of IL-2 mutants in the prior art. On the basis of reducing toxicity, the frequency of administration can be greatly reduced during clinical practice, thereby improving patient compliance with medication.

The modifications in the peptide conjugates of the present invention serve to shield the peptides in peripheral blood circulation, thereby preventing the peptides from activating immune cells such as CD8⁺ T cells and NK cells in the peripheral blood. Therefore, any modification that can achieve the above technical effect is applicable to this application. In a preferred embodiment, the modification includes a polymer or a fatty acid. In a preferred embodiment, the polymer includes PEG. Any PEG capable of achieving the technical effect of the above modification is applicable to the present invention. In a preferred embodiment, the PEG is pyridylthio-PEG with a molecular weight of 9 kDa to 11 kDa, specifically 9 kDa, 10 kDa, or 11 kDa.

Fatty acids are chemical molecules composed of hydrocarbon chains, typically with 6-22 carbon atoms, terminated by a carboxyl group. For the present invention, various fatty acid derivatives capable of conjugating with the amino acid represented by X are also considered to be fatty acids. Fatty acids and their derivatives constitute major lipid components and exhibit hydrophobicity. The length and saturation of the hydrocarbon chain vary from fatty acids to fatty acids, which determines the relevant physical properties. The types of fatty acids include unsaturated fatty acids (polyunsaturated and monounsaturated) and saturated fatty acids-the latter being hydrogen-saturated and predominantly featuring even-numbered straight hydrocarbon chains.

In a preferred embodiment, the fatty acid is an unsaturated or saturated fatty acid selected from those with 6-22 carbon atoms. In a preferred embodiment, the saturated fatty acid is selected from any one of the followings:
17-carboxyheptadecanoic acid,
19-carboxynonadecanoic acid,
4-[N-(2-carboxyethyl)-N-(15-carboxypentadecanoyl)aminomethyl]benzoic acid, or
[2-(2-{2-[2-(2-{2-[4-(17-carboxyheptadecanoylamino)-4(S)-carboxybutyrylamino]ethoxy}etho xy)acetylamino]ethoxy}ethoxy)acetic acid].

The conjugation method of the present invention is not limited, and any chemical group that can be decoupled in the tumor microenvironment is applicable to the present invention. In a preferred embodiment, residue X at position 14 and/or 39 contains a sulfhydryl side chain, enabling bond formation specifically decomposable in the tumor microenvironment. In a preferred embodiment, the modifier is coupled to the sulfhydryl side chain via a disulfide bond, and the disulfide bond of the conjugate is cleaved by tumor-specific enzymes (e.g., redectases), so that the polypeptide in the conjugate is released and exerts its effect. In a preferred embodiment, the above-mentioned modifier is coupled to the sulfhydryl side chain on the cysteine at position 14 and/or 39 of the polypeptide with a disulfide bond through mercaptopyridine. Any modifier capable of the above-mentioned coupling is applicable to the present application. In a preferred embodiment, the modifier includes PEG. There is no particular limitation on the specific type of any amino acid having a sulfhydryl side chain. In a preferred embodiment, the amino acid represented by X at position 14 and/or position 39 of the polypeptide includes cysteine.

In a third typical embodiment of the present application, a DNA molecule is provided, which encodes the above polypeptide or its counterpart within the conjugate.

In a fourth typical embodiment of the present application, a recombinant plasmid is provided, wherein the recombinant plasmid is connected with the above-mentioned DNA molecule.

In a fifth typical embodiment of the present application, a host cell is provided, wherein the recombinant plasmid is transformed. The above-mentioned host cell can be used to replicate the recombinant plasmid in the host cell, and the DNA molecules carried on the recombinant plasmid can also be transcribed and translated to obtain a large number of polypeptides.

In a sixth typical embodiment of the present application, there is provided a use of the above-mentioned polypeptide or polypeptide conjugate in the preparation of a drug for specifically activating CD8+ T cells and/or NK cells in the tumor microenvironment.

Due to steric hindrance from PEG, the polypeptide conjugate is blocked from binding to the β subunit, thereby avoiding systemic toxicity caused by activation of immune cells (NK cells, CD8⁺ T cells) in peripheral circulation. Within the tumor microenvironment, the disulfide bond in the polypeptide conjugate can be decoupled under the action of tumor-specific GSH action, and exert its effect. This enables the accumulation of the conjugate specifically within the tumor environment, thereby reducing the minimum dosage of the therapeutic window. The use of the peptide conjugate described in the present invention significantly decreases the original high frequency of daily dosing, and reduces the amount of each dose, providing a positive impact on the tumor treatment process.

In a preferred embodiment, the above use includes use in the preparation of a drug for promoting immunity.

In a preferred embodiment, the above use includes use in the preparation of a drug for preventing and/or treating solid tumors and hematological malignancies.

In a preferred embodiment, the solid tumor is bladder cancer, bone cancer, brain cancer, breast cancer, colorectal cancer, esophageal cancer, eye cancer, head and neck cancer, kidney cancer, lung cancer, melanoma, ovarian cancer, pancreatic cancer, or prostate cancer.

In a preferred embodiment, the hematological malignancy is chronic lymphocytic leukemia (CLL), small lymphocytic lymphoma (SLL), follicular lymphoma (FL), diffuse large B-cell lymphoma (DLBCL), mantle cell lymphoma (MCL), Waldenström macroglobulinemia, multiple myeloma, extranodal marginal zone B-cell lymphoma, nodal marginal zone B-cell lymphoma, Burkitt lymphoma, non-Burkitt high-grade B-cell lymphoma, primary mediastinal B-cell lymphoma (PMBL), immunoblastic large cell lymphoma, precursor B-lymphoblastic lymphoma, B-cell prolymphocytic leukemia, lymphoplasmacytic lymphoma, splenic marginal zone lymphoma, plasma cell myeloma, plasmacytoma, mediastinal (thymic) large B-cell lymphoma, intravascular large B-cell lymphoma, primary effusion lymphoma, or lymphomatoid granulomatosis.

The following describes the present application in further detail with reference to specific embodiments. These embodiments should not be construed as limiting the scope of protection claimed herein.

### Example 1: Design and screening of polypeptide

To achieve the goal of reducing the toxicity of IL-2 mutants in the prior art in the peripheral blood environment and enabling their functionality in the tumor microenvironment, two polyethylene glycol (PEG) molecules were conjugated to IL-2 through disulfide bonds at the interface between IL-2 and its β receptor. PEG is a hydrophilic, low-immunogenic biocompatible polymer. On the one hand, the large steric hindrance of PEG is used to block the binding of CD122. On the other hand, the disulfide bond can be cleaved in environments with high glutathione (GSH) levels to achieve specific release in the tumor microenvironment and exert activity.

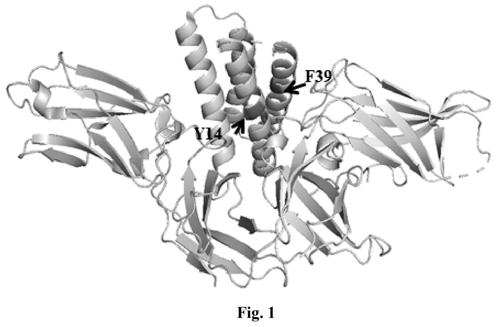

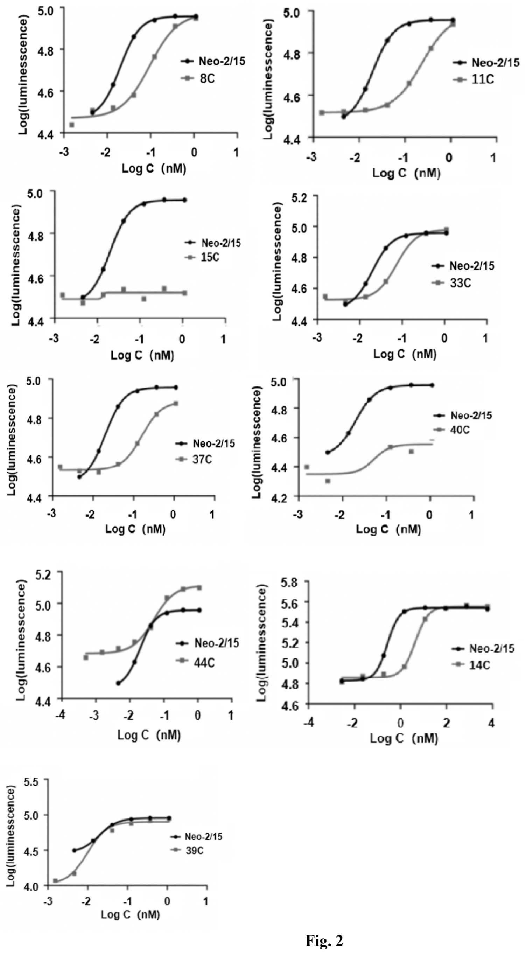

Based on the spatial structure of IL-2 and the β receptor, 3-5 candidate sites were selected for cysteine mutation in each of the two α-helices facing CD122. The cell proliferation activity of CTLL2 after mutation was compared with that of the IL-2 mutant (Neo-2/15) in prior art. The site at position 14 with the spatial site facing the receptor binding surface and the site at position 39 with less effect on cell activity were selected. Finally, these two sites were selected as mutation sites for subsequent experiments (Figure 1-2).

### Example 2: Construction of dual-PEG conjugated IL-2 Mutant

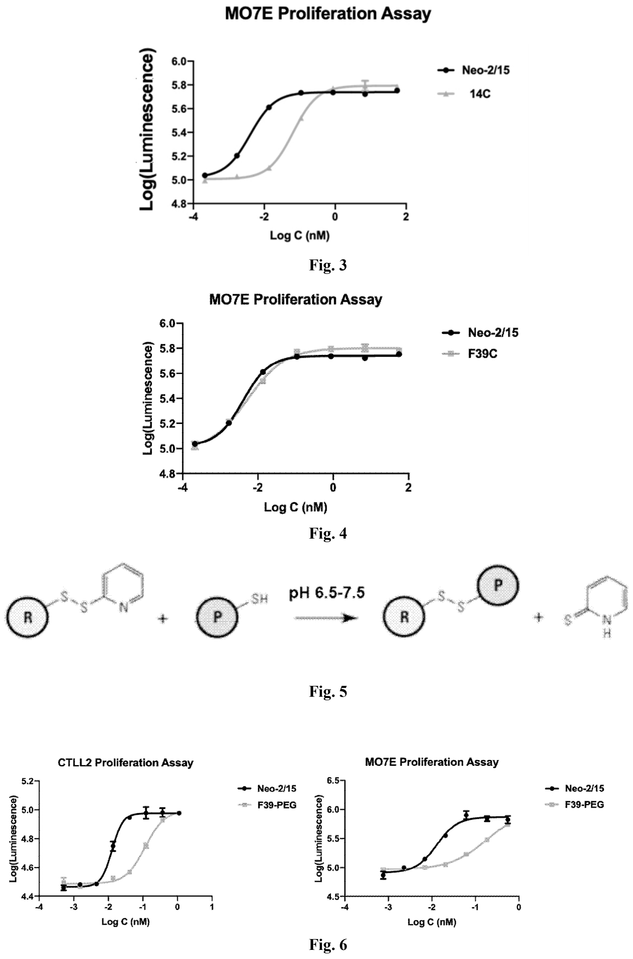

The 14th or 39th position of SEQ ID NO: 1 was substituted with cysteine to obtain polypeptides (14C) and polypeptide (39C), and the effects of the mutations on their function activity were evaluated in CTLL2 and MO7E cells. As can be seen from Figures 3-4, polypeptide (39C) mutant had minimal effect on the functional activity in both CTLL2 and MO7E cells. In contrast, the proliferative activity of polypeptide (14C) mutant was approximately 100-fold weaker than that of Neo-2/15 in both CTLL2 and MO7E cells.

Subsequently, the thiol group of cysteine reacts with the pyridylthio group of OPSS-PEG to form a disulfide bond, thereby completing the conjugation (Figure 5). In Figure 5, R represents a polypeptide containing cysteine, and P represents a PEG with a thiol group. The single mutant was successfully conjugated with a single 10kD PEG (peptide (39C)-PEG and peptide (14C)-PEG), and their activity was verified on CTLL2 and MO7E cells. The results showed that the blocking effect of peptide (39C)-PEG was about 10 times weaker than that of Neo-2/15 (Figure 6).

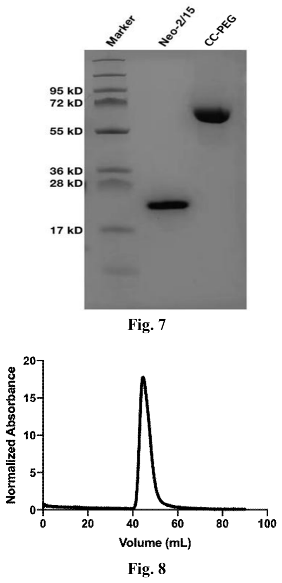

Through point mutation, we successfully constructed the expression plasmid of polypeptide 14C+39C (hereinafter referred to as CC). After transformation into BL21 expression strain, cultures was grown at 37°C until OD600=0.6-0.8 and then induced at 25°C. After high pressure or ultrasonic crushing, supernatant was purified by nickel-affinity chromatography. The eluate was treated with TCEP, concentrated, and subjected to size-exclusion chromatography (DPBS, pH 7.5) to obtain the F-monomer. The concentrated CC was incubated with 10 molar equivalents of OPSS-PEG (10 kDa) at 37° C for 2-3 h. Unreacted OPSS-PEG was removed by anion-exchange chromatography (Tris, pH 9.0), yielding purified CC-PEG conjugate (Figs. 7-8). Final buffer exchange into DPBS was performed.

### Example 3: Validation of CC-PEG Disassociation under GSH Conditions

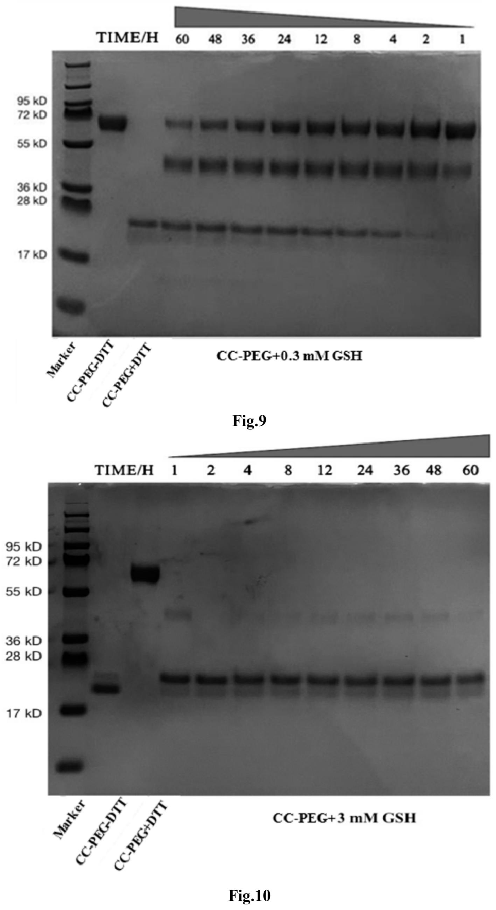

After CC-PEG was incubated with 0.3 mM or 3 mM GSH at 37°C for different times (1h, 2h, 4h, 8h, 12h, 24h, 36h, 48h, 60h), non-reducing SDS-PAGE was performed to verify the decoupling As shown in Fig. 9, under the action of 0.3 mM GSH, CC coupled with dual-PEG can first rapidly released one PEG moiety, and then simultaneously release both PEG groups over time. Maximum decoupling effects was achieved within 8-12 h, and the ratio of CC-PEG with any one PEG removed to CC-PEG with two PEGs removed) is approximately 2:1.

Under higher GSH concentration (Fig. 10, 3 mM), CC-PEG underwent rapid and complete disassociation, with the final product consisting predominantly of fully deconjugated polypeptide and minimal mono-PEGylated species.

Under the action of higher concentrations of GSH (Figure 10, 3 mM GSH), CC-PEG can quickly achieve complete decoupling and thus exert its function. The final decoupling products are mainly peptide without two PEGs, with only a very small amount of peptides with one PEG.

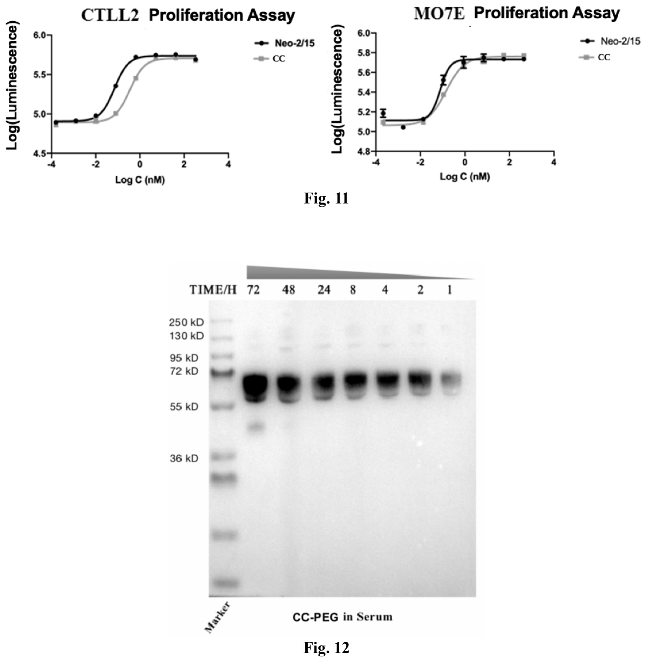

Additionally, the proliferative activity of decoupled CC on CTLL-2 and MO7e cells was tested. As shown in Fig. 11, it can be seen that the decoupled CC has little activity effect on both cells, and can still maintain immune cell activation activity similar to that of Neo-2/15 in tumor tissues.

### Example 4: Plasma Stability Validation of CC-PEG

CC-PEG was incubated in serum at 37°C for different time (1h, 2h, 4h, 8h, 24h, 48h, 72h). Western Blot was used to detect the state of the protein in plasma. The results showed that there was no premature release of CC-PEG in the blood within a certain period of time, greatly avoiding the systemic toxic effects caused by highly active Neo-2/15 (Figure 12).

### Example 5: Cell Function Assay

Based on these experiments, we test the proliferative effects of Neo-2/15, CC-PEG, and the protein obtained by co-incubating CC-PEG with GSH (to simulate the in vivo GSH environment) on CTLL-2 cells expressing trimeric receptors and MO7e cells expressing dimeric receptors.

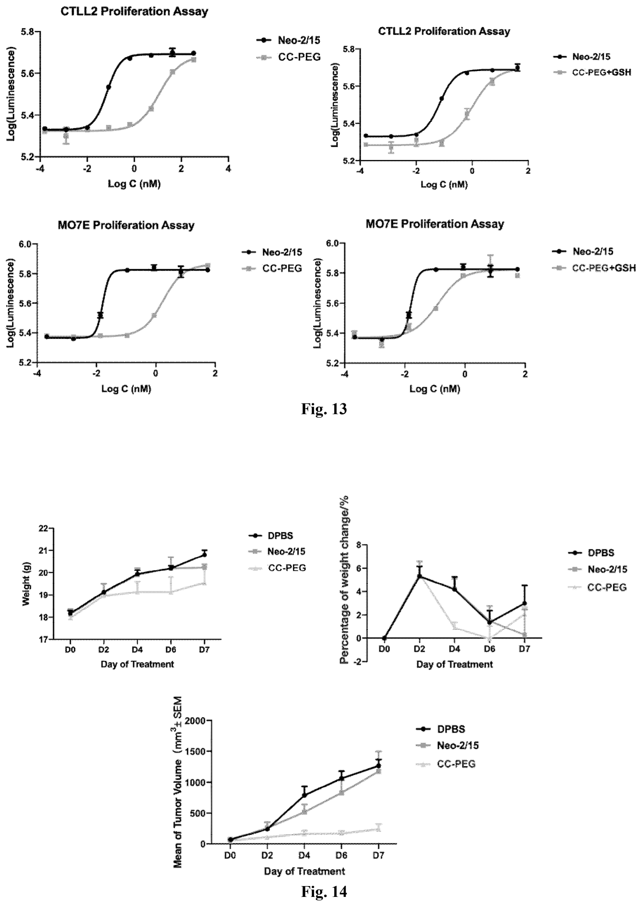

After incubating CTLL2 cells with serially diluted protein concentrations for 18 hours, readings were directly measured using CellTiter-Glo. The results, as shown in Figure 13, demonstrated that compared to Neo-2/15, the proliferative activity of CC-PEG on CTLL2 cells decreased by approximately 170-fold. In contrast, CC obtained after GSH treatment showed only a 14-fold reduction in proliferative activity compared to Neo-2/15, maintaining a similar effect on immune cell proliferation as Neo-2/15. Similarly, after incubating MO7E cells pre-seeded in 96-well plates with serially diluted protein concentrations for 3 days, the results in Figure 13 showed that compared to Neo-2/15, the proliferative activity of CC-PEG on MO7E cells decreased by approximately 110-fold. In contrast, CC obtained after GSH treatment exhibited only a 7-fold reduction in proliferative activity compared to Neo-2/15, still maintaining a similar effect on immune cell proliferation as Neo-2/15.

In summary, CC-PEG effectively blocked activity blocking and restored activity after GSH treatment on both MO7E and CTLL2 cell lines. Due to preserved lack of CD25 binding affinity inherited from Neo-2/15, the reactivated CC preferentially activates cells expressing dimeric receptors when functionally restored. Comparing the overall effects on CTLL2 and MO7E, since CC retained the property of neo-2/15 of not binding to CD25, CC-PEG ultimately tended to act on cells expressing dimeric receptors.

### Example 6: Pharmacokinetics of CC-PEG in Mice

Female C57BL/6 mice were randomly assigned to two groups (6 mice per group) and injected with equivalent doses of Neo-2/15 or CC-PEG via tail vein. Eye blood samples were collected at various time points. The serum concentrations of the compounds were quantified by ELISA, and the data were processed by GraphPad Prism software. The results are shown in Table 1, revealed that the half-life of CC-PEG was significantly prolonged compared to that of Neo-2/15 (Table 1).

Female C57BL mice were randomly divided into two groups (6 mice/group) and injected with equal doses of Neo-2/15 or CC-PEG via the tail vein. Eye blood was collected at different time points. ELISA was used to detect the concentration of Neo-2/15 and CC-PEG in each sample, and the data were processed by GraphPad Prism. The results are shown in Table 1, showing that the in vivo half-life of CC-PEG was greatly prolonged compared with that of Neo-2/15.

**Table 1: Pharmacokinetic Parameters of CC-PEG in Mice**

| | Neo-2/15 | CC-PEG |
|---|---|---|
| T_{1/2} | 0.67h | 10.4h |
| Cₘₐₓ | 50.5ug/ml | 15.12 ug/ml |

### Example 7: Efficacy Evaluation of CC-PEG in Mice

Female C57BL/6J mice were randomized into PBS, Neo-2/15, and CC-PEG groups (3 mice per group), followed by subcutaneous inoculation with B₁₆F₁₀SIY melanoma cells (a murine melanoma cell line widely used in basic and clinical research on cancer and tumors). When tumor size reached a volume of about 50 mm³, 100 µL PBS, 0.15 mg/kg Neo-2/15, or CC-PEG were administered by intraperitoneal injection, and the day was recorded as D0. Thereafter, the Neo-2/15 group received daily injections from Day 0 to Day 5, whereas PBS and CC-PEG groups were dosed twice (Day 0 and Day 4). During this period, the changes in tumor volumes were recorded every two days. When the tumor volume of mice in PBS group exceeded 1000 mm³ , the mice were euthanized, and the whole blood, spleen, and tumor tissue were collected for lymphocyte composition analysis.

As shown in Fig. 14, compared to the PBS group, continuous administration of Neo-2/15 at a high frequency can play a role in delaying tumor growth in a short period of time, whereas CC-PEG, which is administered only twice, can significantly inhibit tumor growth on the basis of greatly reducing the dosing frequency.

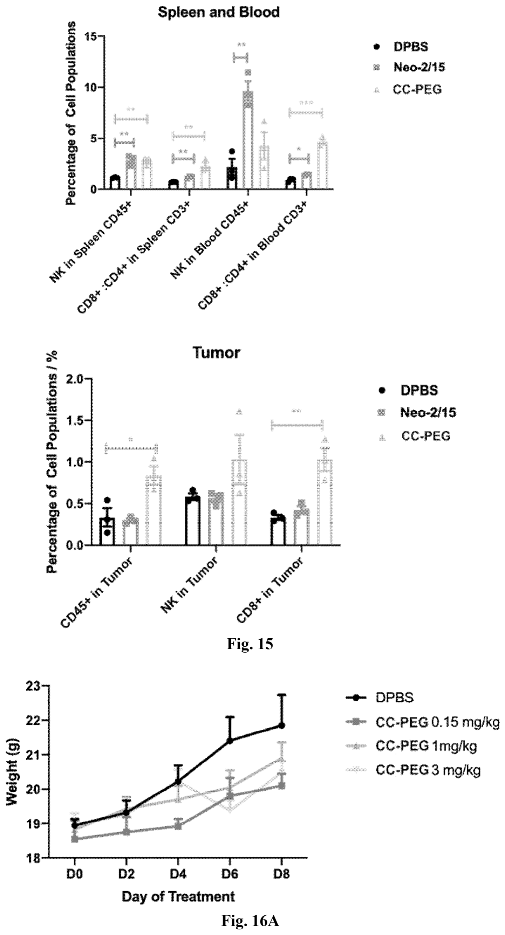

The flow cytometry results (Fig. 15) demonstrated that CC-PEG markedly increased the proportion of CD8⁺/CD4⁺ T cells in peripheral blood and spleen, which play a major role in adaptive immunity, while multiple administrations of Neo-2/15 significantly increased the proportion of NK cells in peripheral blood. As a member of the innate immune system, the substantial increase in NK cells in peripheral blood may be closely related to the induction of inflammatory response. In the flow cytometry of tumor tissue, we detected that CC-PEG can enhance the infiltration of lymphocytes in tumors and increase the proportion of NK cells, thereby reducing the proportion of NK cells in peripheral blood and reducing the possibility of inducing inflammation.

### Example 8: Analysis of dose-dependent anti-tumor efficacy of CC-PEG in mice

Female C57BL/6J mice were randomized into PBS control and three CC-PEG groups with different dosages (0.15 mg/kg, 1 mg/kg, 3 mg/kg; n=4 per group), followed by subcutaneous inoculation with B₁₆F₁₀SIY melanoma cells. When tumor size reached approximately 50 mm³ , the drugs were administered via intraperitoneal injection. Four days later, a second administration was performed in the same way, and the tumor volume of the mice was recorded every two days. When the tumor volume of the mice in the PBS group was greater than 1000mm3, the mice were euthanized.

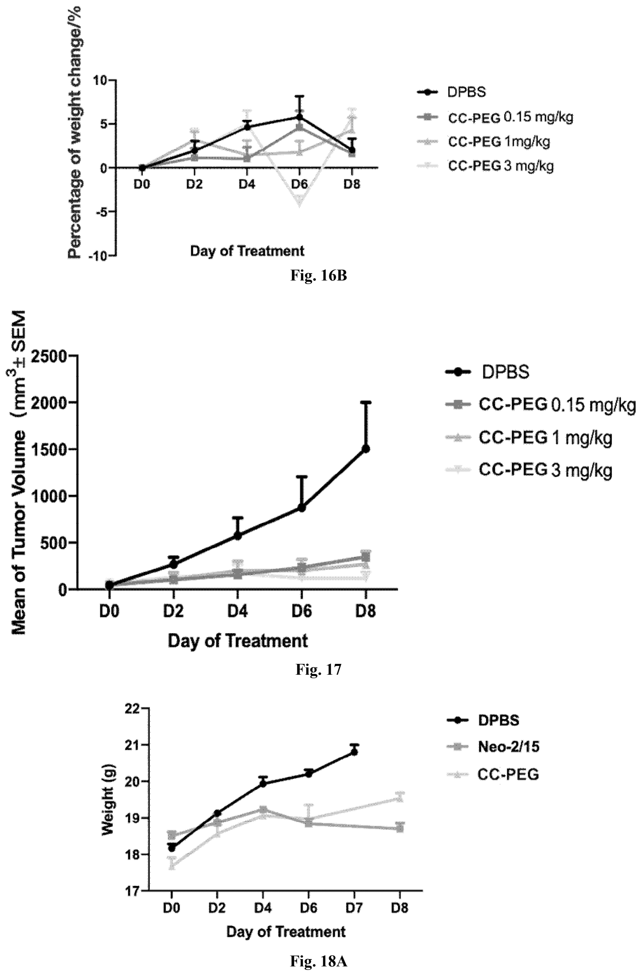

As shown in Figs. 16A, 16B, and 17, compared with the DPBS control group, the CC-PEG groups at each dose demonstrated significant tumor suppression, with a clear dose-dependent enhancement of the antitumor effect as the dosage increased. Body weight measure indicated that CC-PEG treatment below 1 mg/kg has basically no effect on mouse body weight, and all show a slowing rising trend. Administration of 3 mg/kg will cause a brief decline in body weight, and recover within two days. Therefore, CC-PEG is capable of achieving significant antitumor effects as a monotherapy within a relatively wide therapeutic window.

### Example 9: Toxicity Assessment of CC-PEG in Mice

Female C57BL/6J mice were randomly divided into three groups (n=3 per group): PBS, Neo-2/15, and CC-PEG. All mice received subcutaneous inoculations of B₁₆F₁₀SIY melanoma cells. When the tumor size reached a volume of about 50 mm³, 100 µL PBS, 1 mg/kg Neo-2/15 or 1mg/kg CC-PEG were administered by intraperitoneal injection, and the day was recorded as D0. Thereafter, the Neo-2/15 group was continuously administered from D0 to D5, and the PBS group and CC-PEG group were administered twice on D0 and D4, respectively. The changes in the tumor volume of mice were recorded every two days, and eye blood was collected 6 hours after the second administration of CC-PEG on D4 and 6 hours after the continuous administration of Neo-2/15 on D5 and D6. When the tumor volume of mice in the PBS group was greater than 1000 mm³, the mice were euthanized.

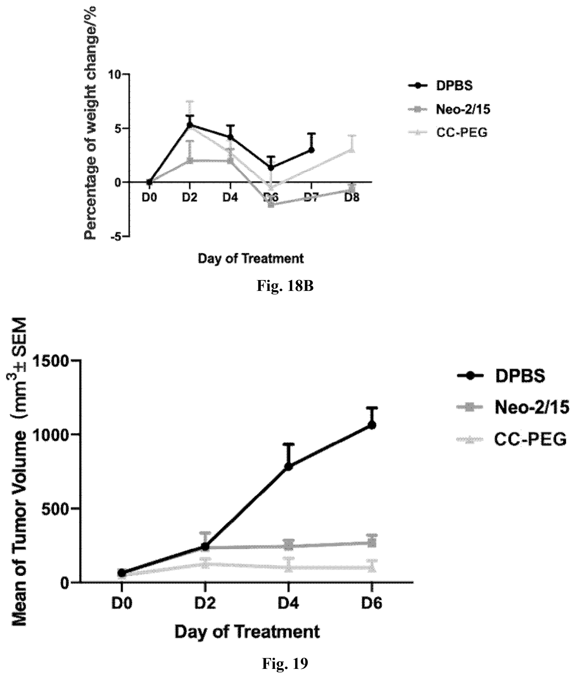

As shown in Figs. 18A, 18B, and 19, both continuous Neo-2/15 (1 mg/kg) and biweekly CC-PEG significantly suppressed tumor growth, with CC-PEG demonstrating superior efficacy. Notably, Neo-2/15 administration caused progressive body weight decline starting Day 4.

As shown in Figures 18A, 18B and 19, continuous administration of 1mg/kg Neo-2/15 and Q4D administration of 1mg/kg CC-PEG can significantly inhibit tumor growth, but the tumor growth curve shows that CC-PEG is more effective than Neo-2/15. Under these conditions, the body weight of mice decreased after continuous administration of Neo-2/15 on D4, and continued to decrease with subsequent administration.

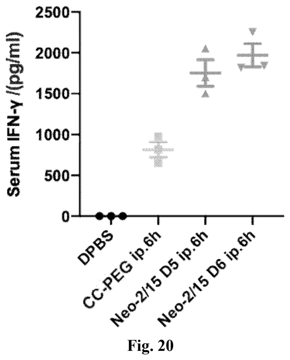

ELISA detection of plasma cytokine levels (as shown in Figure 20) revealed no IFN-γ production in the DPBS group. Between the two treatment groups, CC-PEG demonstrated superior antitumor efficacy compared to Neo-2/15, and continuous administration of Neo-2/15 induced higher IFN-γ production than CC-PEG, which may have caused a decrease in the body weight of mice, suggesting that CC-PEG is safer than Neo-2/15.

From the above description, it can be seen that the above-mentioned embodiments of the present invention achieve the following technical effects: the polypeptides (polypeptide (14C), polypeptide (39C), CC) and polypeptide conjugates (CC-PEG) in the present application have the characteristics of not binding to CD25 and weakening binding to CD122. And the polypeptide conjugate (CC-PEG) functions specifically with tumor tissue, significantly inhibit tumor growth. This reduces systemic toxicity associated with IL-2 variants of the prior art in systemic immunity, increases the maximum tolerated dose within the therapeutic window, and provides a wider range of choices of dosing options in clinical trials. In addition, the peptide conjugate (CC-PEG) demonstrates an extended half-life in mice, substantially reducing the frequency of administration. Due to its ability to accumulate extensively in tumor tissues, the required dosage is also significantly lowered. Thus, the peptides and peptide conjugates of the present invention provide significant therapeutic advantages for tumor treatment.

These embodiments represent preferred implementations but do not limit the invention. Practitioners may make various modifications, equivalent substitutions, and improvements within the spirit and principles of this invention, all encompassed within its protective scope.

## Claims

1. A polypeptide, **characterized in that** the polypeptide comprises the amino acid sequence set forth in SEQ ID NO: 1, wherein the amino acid represented by X at position 14 and/or position 39 satisfies the followings:
(i) capable of conjugating with a modifier; and
(ii) capable of decoupling from said modifier in a tumor microenvironment to release the polypeptide;
wherein the binding capability of the polypeptide without conjugation to said modifier to CD122 is designated as a first binding capability,
the binding capability of the polypeptide conjugated to said modifier to CD122 is designated as a second binding capability,
and the ratio of the first binding capability to the second binding capability is designated as n, wherein n ≥ 5.

2. The polypeptide according to claim 1, **characterized in that** the amino acid represented by X at position 14 and/or position 39 is any amino acid contains a sulfhydryl side chain;
preferably, the amino acid represented by X at position 14 and/or position 39 forms an intramolecular covalent linkage directly or via a linker;
preferably, the linker comprises mercaptopyridine;
preferably, the amino acid represented by X at position 14 and/or position 39 is cysteine.

3. A polypeptide conjugate, **characterized by** comprising:
a polypeptide having the amino acid sequence of SEQ ID NO: 1, wherein X at position 14 and/or 39 is an amino acid capable of conjugating with a modifier and decoupling from said modifier in a tumor microenvironment to release the polypeptide;
wherein the binding capability of the polypeptide without conjugation to said modifier to CD122 is designated as a first binding capability, the binding capability of the polypeptide conjugated to said modifier to CD122 is designated as a second binding capability, and the ratio of the first binding capability to the second binding capability is designated as n, wherein n ≥ 5.

4. The polypeptide conjugate according to claim 3, **characterized in that** the modifier comprises a polymer or a fatty acid;
preferably, the polymer comprises a PEG;
preferably, the fatty acid comprises an unsaturated fatty acid or saturated fatty acid;
preferably, the unsaturated or saturated fatty acid is selected from fatty acids with 6 to 22 carbon atoms;
preferably, the saturated fatty acid is selected from any one of:
17-carboxyheptadecanoic acid,
19-carboxynonadecanoic acid,
4-[N-(2-carboxyethyl)-N-(15-carboxypentadecanoyl)aminomethyl]benzoic acid, or
[2-(2-{2-[2-(2-{2-[4-(17-carboxyheptadecanoylamino)-4(S)-carboxybutyrylamino]ethoxy }ethoxy)acetylamino]ethoxy}ethoxy)acetic acid].

5. The polypeptide conjugate according to claim 4, **characterized in that** the amino acid represented by X at position 14 and/or 39 of the polypeptide is any amino acid containing a sulfhydryl side chain;
preferably, the modifier is conjugated via a disulfide bond to the sulfhydryl side chain of the amino acid at position 14 and/or 39;
preferably, the modifier is conjugated via mercaptopyridine to the sulfhydryl side chain through a disulfide bond;
preferably, the modifier is PEG;
preferably, the amino acid represented by X at position 14 and/or 39 is cysteine.

6. A DNA molecule, **characterized in that** it encodes:
(i) the polypeptide according to claim 1 or 2; or
(ii) the polypeptide from the polypeptide conjugate according to any one of claims 3 to 5.

7. A recombinant plasmid, **characterized in that** it is ligated to the DNA molecule according to claim

8. A host cell, **characterized in that** it is transformed with the recombinant plasmid according to claim 7.

9. Use of the polypeptide according to claim 1 or 2, or the polypeptide conjugate according to any one of claims 3 to 5 in the preparation of a drug for specifically activating CD8+ T cells and/or NK cells a tumor microenvironment.

10. The use according to claim 9, **characterized in that** said use comprises use in the preparation of a medicament for promoting immunity.

11. The use according to claim 10, **characterized in that** said use comprises use in the preparation of a medicament for preventing and/or treating solid tumors or hematological malignancies;
preferably, said solid tumor is bladder cancer, bone cancer, brain cancer, breast cancer, colorectal cancer, esophageal cancer, eye cancer, head and neck cancer, kidney cancer, lung cancer, melanoma, ovarian cancer, pancreatic cancer or prostate cancer;
preferably, said hematological malignancy is chronic lymphocytic leukemia (CLL), small lymphocytic lymphoma (SLL), follicular lymphoma (FL), diffuse large B-cell lymphoma (DLBCL), mantle cell lymphoma (MCL), Waldenström macroglobulinemia, multiple myeloma, extranodal marginal zone B-cell lymphoma, nodal marginal zone B-cell lymphoma, Burkitt lymphoma, non-Burkitt high-grade B-cell lymphoma, primary mediastinal B-cell lymphoma (PMBL), immunoblastic large cell lymphoma, precursor B-lymphoblastic lymphoma, B-cell prolymphocytic leukemia, lymphoplasmacytic lymphoma, splenic marginal zone lymphoma, plasma cell myeloma, plasmacytoma, mediastinal (thymic) large B-cell lymphoma, intravascular large B-cell lymphoma, primary effusion lymphoma or lymphomatoid granulomatosis.
